# EUROPÄISCHE PATENTSCHRIFT

(11) **EP 4 117 589 B1**
(45) Veröffentlichungstag und Bekanntmachung des Hinweises auf die Patenterteilung: **16.07.2025**
(21) Anmeldenummer: 21712953.5
(22) Anmeldetag: 09.03.2021
(51) Int. Cl.: A61F 5/01, A61B 5/103, A61B 5/00

(54) **ORTHOPÄDISCHES FEEDBACK-SYSTEM UND FEEDBACK-VERFAHREN**
ORTHOPAEDIC FEEDBACK SYSTEM AND FEEDBACK METHOD
SYSTÈME DE RÉTROACTION ORTHOPÉDIQUE ET PROCÉDÉ DE RÉTROACTION

(30) Priorität: 09.03.2020 DE 102020001487
(43) Veröffentlichungstag der Anmeldung: 18.01.2023
(73) Patentinhaber: Noz Leipzig Forschung Technik GbR, 04109 Leipzig (DE)
(72) Erfinder: SRUGIES, Stefan, 04838 Doberschütz (DE)
(74) Vertreter: Sperling, Thomas
(86) Internationale Anmeldenummer: PCT/DE2021/100239
(87) Internationale Veröffentlichungsnummer: WO 2021/180276

(56) Entgegenhaltungen:
- DE-A1- 102011 012 458
- US-A1- 2013 151 699
- US-A1- 2018 332 933
- US-B1- 6 693 516

## Beschreibung

Die Erfindungen betrifft ein orthopädisches Feedbacksystem in Verbindung mit einem orthopädischen Stützsystem. Ferner betrifft die Erfindung ein Feedback-Verfahren.

Orthopädische Stützvorrichtungen, sogenannte Orthesen, sind körpernahe, das heißt an einem Köperteil oder eine Körperregion angebrachte Hilfsmittel, welche eine Beweglichkeit und Stabilität von Körperteilen oder Körperregionen gewährleisten sollen. Orthesen sind für verschiedene Körperteile oder Körperregionen verfügbar. Als Beispiele sind Knie-, Fuß-, Hüft-, Ellenbogen- oder Rückenorthesen zu nennen. Orthesen werden zur Stabilisierung, Entlastung, Ruhigstellung, Führung oder Korrektur von Gliedmaßen oder des Rumpfes eingesetzt und dienen außerdem zum Ausgleich von Funktionsausfällen der Extremitäten oder der Wirbelsäule. Somit können Orthesen für therapeutische Maßnahmen zum Anlernen oder zur Wiedererlangung von natürlichen Bewegungsabläufen eingesetzt werden. Ein Therapieansatz besteht darin, einen Trainingseffekt durch eine orthesengestützte Kombination von Bewegungswiederholungen und Belastungssteigerungen zu erreichen. Dies erfordert üblicherweise ein Mitwirken eines Patienten, welcher eine Belastungsgrenze sensorisch durch Schmerz erfassen kann. Es treten jedoch Krankheitsbilder wie Schlaganfall, Multiple Sklerose, inkomplette Querschnittlähmung, Morbus Parkinson, Schädelhirntraumen, sensorische Funktionsstörungen (z.B. Polyneuropathie) oder Postpoliosyndrome auf, bei welchen sensorische Empfindungen, wie Tastsinn und Schmerz, beeinträchtigt sind oder nicht mehr wahrgenommen werden können. Fehlt ein sensorisches Input beispielsweise im Bereich der unteren Extremitäten, ist ein adäquates Antworten auf veränderte Situationen im Gang nicht möglich, was zu Minder- oder Überbelastung führt und demzufolge den Heilungs- beziehungsweise Therapiefortschritt ungünstig beeinflusst. Nachteilig ist weiterhin, dass Bewegungsabläufe ohne eine bewusste Reizwahrnehmung bei der Bewegung nicht gefestigt oder nur sehr langsam wiedererlangt werden können. In solchen Fällen ist es bislang Ziel der Physiotherapie und Ergotherapie, sensorischen Input durch Berührungsreize in sensiblen Körperregionen zu geben, um damit Bewegungen zu erleichtern oder auszulösen. Dabei helfen Orthesen fehlende Kraft und verlorengegangene Muskulatur zu ersetzen oder aufzubauen.

Insbesondere Bein- oder Fußorthesen bekannter Art stoßen oft aufgrund einer mangelnden Vorfußbelastung an Ihre Funktionsgrenzen und können den Patienten nicht im gewünschten Umfang unterstützen. Dies führt bei vielen Patienten, welche sensorische Defizite im Bereich der Extremität aufweisen, zu dem Ergebnis, dass das eigene Potenzial, das Potenzial der Therapie und das Potenzial der Hilfsmittelversorgung nicht voll ausgenutzt werden kann. Die Funktionsweise der Sensomotorik wird in der Orthopädietechnik zwar in Form von sensomotorischen Einlagen verwendet und auch mit anderen orthopädietechnischen Produkten kombiniert, allerdings setzt dies eine gewisse Restsensorik beim Patienten voraus. Ohne ein Gefühl im Bein oder Fuß des Patienten ist es nicht möglich, das nötige Feedback zu geben, um gewünschte Muskeln adäquat anzusteuern. Die Patienten entwickeln dadurch Schutzstrategien, die zu Lasten der Ökonomie beim Stehen und Gehen führen.

Aus DE 10 2011 012 458 A1 ist ein Feedback-System bekannt, mit welchem an einer Schuheinlage (Sohle) erfasste Drucksignale für einen Benutzer wahrnehmbar als Feedback-Signal wiedergegeben werden. Die Signalwiedergabe von erfassten Druckkraftwerten erfolgt mittels einem Wiedergabeelement, beispielsweise Smartphone, visuell, akustisch oder durch Vibration. Die Erfassung der Druckkraft erfolgt undifferenziert, wobei eine erfasste Drucckraft als Feedback in Form eines einfachen Signals wiedergegeben wird.

Es ist daher Aufgabe der Erfindung, ein orthopädisches Feedback-System zur therapeutischen Unterstützung von Patienten mit taktilen und haptischen Empfindungsstörungen vorzuschlagen. Aufgabe ist es weiterhin, eine entsprechendes Feedback-Verfahren vorzuschlagen.

Erfindungsgemäß wird die Aufgabe mit einem System mit den Merkmalen des Anspruchs 1 und einem Verfahren mit den Merkmalen des Anspruchs 10 gelöst. Vorteilhafte Ausgestaltungen und Weiterbildungen können jeweils mit den in untergeordneten Ansprüchen bezeichneten Merkmalen realisiert werden.

Der Erfindung liegt die Idee zugrunde, bei Patienten mit bereichsweise gestörtem taktilen beziehungsweise haptischen Reizempfinden, an einer gestützten Körperregion oder an dem gestützten Körperteil auftretende Druckbelastungen, mittels taktilen beziehungsweise haptischen Reizen in einer sensiblen Körperregion beziehungsweise an einem sensiblen Körperteil des Patienten spürbar zu machen. Auf diese Weise soll ein sensorisches Feedback in Form von taktilen Reizen von an einem orthopädischen Stützsystem und/oder einem mit dem orthopädischen Stützsystem in Eingriff stehenden Körperteil auftretenden Belastungen ermöglicht werden.

Das orthopädisches Feedback-System weist mindestens eine orthopädische Stützvorrichtung auf, welche mindestens ein mit einem ersten Körperteil oder einer ersten Körperregion in Eingriff befindliches Stützelement aufweist. Bei der orthopädischen Stützvorrichtung handelt es sich um eine Orthese oder eine Prothese, welche im Benutzungszustand mit einem Körperteil oder einer Körperregion ein Eingriff steht.

Weiterhin weist das orthopädisches Feedback-System eine mehrere Druckkrafterfassungssensoren aufweisende Druckkrafterfassungseinrichtung auf, welche zur Erfassung von einer auf das erste Körperteil, die erste Körperregion oder das mindestens eine Stützelement wirkende Druckkraft eingerichtet ist. Dabei soll der Begriff Druckkraft als Oberbegriff für verschiedene mechanische Belastungsarten wie Dehnung und Torsion gelten, welche an dem ersten Körperteil, der ersten Körperregion oder dem mindestens einen Stützelement sensorisch erfassbar sind.

Zur Wiedergabe von mit der Druckkrafterfassungseinrichtung erfassten Druckkräften weist das orthopädisches Feedback-System eine mit der Druckkrafterfassungseinrichtung in Kommunikationsverbindung stehende Wiedergabeeinrichtung auf, welche mindestens ein mit einem zweiten Körperteil oder einer zweiten Körperregion kontaktiertes oder von dem zweiten Körperteil oder der zweiten Körperregion in Eingriff befindliches Wiedergabeelement aufweist, welches zur taktilen Wiedergabe eines von der Drucckrafterfassungseinrichtung erfassten Druckkraftsignals an das zweite Körperteil oder die zweite Körperregion eingerichtet ist.

Das Wiedergabeelement, welches zur taktilen Wiedergabe eines von der Druckkrafterfassungseinrichtung erfassten Druckkraftsignals eingerichtet ist, dient zum Bereitstellen eines vom Patienten wahrnehmbaren taktilen Reizes zur Rückkopplung von auf das erste Körperteil, die erste Körperregion oder das mindestens eine Stützelement wirkenden Druckkräften. Im Benutzungszustand ist das Wiedergabeelement mit dem zweiten Körperteil oder der zweiten Körperregion kontaktiert. Dabei können das zweite Körperteil oder die zweiten Körperregion und das Wiedergabeelement ineinandergreifen. Beispielsweise kann das zweite Körperteil oder die zweiten Körperregion das Wiedergabeelement umgreifen. Bei dem zweiten Körperteil oder der zweiten Körperregion handelt es sich um einen sensorisch sensiblen Körperbereich, welcher eine haptische Wahrnehmung von mit dem Wiedergabeelement wiedergegebenen taktilen Reizen ermöglicht. Das Wiedergabeelement ist daher mit einem sensorisch sensiblen Körperbereich kontaktiert.

Gemäß verschiedenen Ausgestaltungen des orthopädisches Feedback-Systems kann vorgesehen sein, dass das Wiedergabeelement der Wiedergabeeinrichtung in eine separate Stützeinrichtung, welche sich von dem zweiten Körperteil oder der zweiten Körperregion in Eingriff befindet, integriert ist. Dabei kann die separate Stützeinrichtung auch als Bestandteil der orthopädischen Stützvorrichtung ausgebildet sein. Es kann ferner vorgesehen sein, dass das Wiedergabeelement in einem Griff einer Gehstütze integriert ist.

Gemäß weiteren verschiedenen Ausgestaltungen des orthopädisches Feedback-Systems kann vorgesehen sein, dass das Wiedergabeelement der Wiedergabeeinrichtung in ein Kleidungstück integriert ist. Ferner kann das Wiedergabeelement der Wiedergabeeinrichtung in einem Armband, einem Brustband oder in eine Rumpfmanschette integriert sein.

Gemäß einer besonders einfachen Ausgestaltung umfasst das Wiedergabeelement mindestens einen Vibrationsaktor, dessen Vibrationsintensität in Abhängigkeit von der Intensität eines erfassten Druckkraftsignals der Druckkrafterfassungseinrichtung steuerbar ist. Erfindungsgemäß weist das Wiedergabeelement eine mehrere Aktoren aufweisende Wiedergabematrix auf, welche eingerichtet ist, ein von der Druckkrafterfassungseinrichtung erfasstes Druckkraftprofil an dem zweiten Körperteil oder die zweite Körperregion wiederzugeben. Die Aktoren zur Wiedergabe von taktilen Reizen sind beabstandet angeordnet. Wiedergabeelemente zur taktilen Wiedergabe eines von der Druckkrafterfassungseinrichtung erfassten Druckkraftsignals können an verschiedenen sensiblen Körperregionen angebracht werden. Vorgesehen sein kann eine Integration des Wiedergabeelements in einem Brustgurt, in einem Büstenhalter, in einem Armband, in einem Halsband oder in einer Halskette, in einem Stirnband oder in eine Brille. Es kann ferner vorgesehen sein, dass Wiedergabeelemente an verschiedenen Positionen am Körper angebracht werden. Gemäß einer Weiterbindung der Erfindung kann eine Integration von Wiedergabeelementen in einem rechten Armband und in einem linken Armband vorgesehen sein, wobei unterschiedliche erfasste Druckkraftsignale zur Wiedergabe an das linke Armband oder an das rechte Armband bereitgestellt werden. Beispielsweise kann ein an einem linken Fuß erfasstes Druckkraftsignal mit der Wiedergabeeinrichtung zur Wiedergabe an ein linkes Armband bereitgestellt werden, wobei die Wiedergabeeinrichtung ein Wiedergabesignal eines an einem rechten Fuß erfassten Druckkraftsignals an ein rechtes Armband bereitgestellt. Entsprechende Kombinationen können für verschiedene weitere Körperregionen oder Körperpositionen vorgesehen werden. Zur Wiedergabe von erfassten Druckkraftsignalen kann weiterhin eine Stimulation via Processus mastoideus mittels hochfrequenter Vibration vorgesehen sein.

Die Kommunikationsverbindung zwischen der Druckkrafterfassungseinrichtung und der Wiedergabeeinrichtung kann drahtlos ausgebildet sein. Als Drahtlosverbindung kann beispielsweise eine Bluetooth-Verbindung vorgesehen sein. Entsprechende Kommunikationsmodule zur Bereitstellung einer Drahtlosverbindung, beispielswiese Bluetooth-Verbindung, sind aus dem Stand der Technik bekannt.

Gemäß einer bevorzugten Ausgestaltung des orthopädisches Feedback-Systems kann die Druckkrafterfassungseinrichtung in Form einer Einlegesohle zur Einlage in einen Schuh ausgebildet sein. Dabei kann die Einlegesohle mehrere flächig beabstandet angeordnete Druckkrafterfassungssensoren aufweisen, welche zur Erfassung eines Druckkraftprofils eingerichtet sind. Weiterhin kann die Druckkrafterfassungseinrichtung zur Einarbeitung auf eine orthopädische Einlage oder zur festen Verbindung mit dem Stützelement ausgebildet sein.

Die Druckkrafterfassungseinrichtung kann eingerichtet sein, die Signale der Druckkrafterfassungssensoren der Einlegesole oder der orthopädischen Einlage als Heat-Map-Diagramm oder topographisches Diagramm zu erfassen, wobei das Wiedergabeelement eingerichtet sein kann, die Informationen eines erfassten Heat-Map-Diagramms oder eines erfassten topographisches Diagramms taktil an dem zweiten Körperteil oder der zweiten Körperregion wiederzugeben.

Die Druckkrafterfassungssensoren der Druckkrafterfassungseinrichtung können an dem mindestens einen Stützelement angeordnet sein. Vorzugsweise erfolgt die Druckkrafterfassung unter der Sohle eines Fußes. Die Druckkrafterfassungssensoren der Drucckrafterfassungseinrichtung sind daher bevorzugt in einer orthopädischen Einlage, beispielsweise Schuheinlage, integriert.

Gemäß verschiedenen Ausgestaltungen des orthopädisches Feedback-System kann die Druckkrafterfassungseinrichtung mindestens vier einzelne Druckkrafterfassungssensoren aufweisen. Bevorzugt kann die Druckkrafterfassungseinrichtung mehr als 100 einzelne Drucckrafterfassungssensoren aufweisen. Zur Erstellung von detaillierten Heat-Map-Diagrammen oder topographischen Diagrammen, welche eine Analyse komplexer Druckkraftbelastungen ermöglichen, kann die Druckkrafterfassungseinrichtung mehr als 1000 einzelne Druckkrafterfassungssensoren aufweisen. Dabei können die Drucckraftsensoren in eine Flächenmatrix angeordnet sein.

Die Erfindung umfasst ferner ein Feedback-Verfahren, welches bevorzugt mit dem erfindungsgemäßen orthopädischen Feedback-System durchführbar ist. Bei dem Feedback-Verfahren wird eine an oder auf ein erstes Körperteil, eine erste Körperregion und/oder ein Stützelement für das erste Körperteil oder die erste Körperregion wirkende Druckkraft erfasst und die erfasste Druckkraft mittels eines Wiedergabeelements an einem zweiten Körperteil oder eine zweite Körperregion mit einem taktilen Reiz wiedergegeben.

Erfindungsgemäß wird die Druckkraft an mehreren Druckkrafterfassungspositionen an dem ersten Körperteil, der ersten Körperregion und/oder dem Stützelement für das erste Körperteil oder die erste Körperregion als Druckkraftprofil, Heat-Map-Diagramm oder topographisches Diagramm erfasst, wobei die Informationen des Druckkraftprofils, des Heat-Map-Diagramms oder des topographischen Diagramms mit taktilen Reizen an mehreren Wiedergabepositionen an dem zweiten Körperteil oder der zweiten Körperregion wiedergegeben werden.

Es kann vorgesehen werden, dass eine Intensität des taktilen Reizes, welcher an dem zweiten Körperteil oder der zweiten Körperregion wiedergegeben wird, mit einer Intensität der an dem ersten Körperteil, der ersten Körperregion oder dem Stützelement erfassten Druckkraft korreliert. Ferner kann vorgesehen werden, dass eine Intensität einer erfassten Druckkraft im Verhältnis zur tatsächlichen Belastung durch die Intensität des taktilen Reizes stärker oder schwächer wiedergegeben wird.

Weiterhin kann vorgesehen werden, dass der taktile Reiz bei Überschreiten und/oder Unterschreiten eines vorgegebenen Schwellenwertes einer erfassten Druckkraft wiedergegeben wird. So kann vorgesehen werden, dass ein taktiler Reiz an dem zweiten Körperteil oder der zweiten Körperregion bei Erfassung einer Überschreitung einer vorgegebenen Druckkraft wiedergegeben wird. Ferner kann vorgesehen werden, dass ein taktiler Reiz an dem zweiten Körperteil oder der zweiten Körperregion bei Erfassung einer Unterschreitung einer vorgegebenen Druckkraft wiedergegeben wird. Gemäß einer dritten Variante kann ein taktiler Reiz an dem zweiten Körperteil oder der zweiten Körperregion bei Erfassung einer Überschreitung und einer Unterschreitung einer vorgegebenen Druckkraft wiedergegeben werden. Durch die Anwendung von Schwellenwerten der Druckkraft zum Auslösen des taktilen Reizes können therapeutische Maßnahmen realisiert werden, um einem Patienten eine Unterbelastung und/oder Überbelastung zu signalisieren.

Gemäß verschiedenen Ausführungsvarianten des Feedback-Verfahrens kann vorgesehen werden, dass bei Überschreiten und/oder Unterschreiten eines vorgegebenen Schwellenwertes einer erfassten Druckkraft ein vorgegebener taktiler Reiz wiedergegeben wird. Dabei unterscheidet sich der vorgegebene taktile Reiz, welcher bei Überschreiten und/oder Unterschreiten eines vorgegebenen Schwellenwertes einer erfassten Druckkraft ausgelöst wird, von den taktilen Reizen, welche in ihrer Intensität und ihrem Wiedergabemuster mit der erfassten Druckkraft korrelieren. Vorzugsweise kann der vorgegebene taktile Reiz als periodisches Vibrationssignal für eine vorgegebene Zeitdauer wiedergegeben werden. Durch die Wiedergabe von vorgegeben taktilen Reizen können weitere therapeutische Maßnahmen realisiert werden, in dem vorgegebene taktile Reize mit vorgegebenen Wiedergabemuster beispielsweise als Warnsignal zur Signalisierung einer Überbelastung wiedergegeben werden.

Taktile Reize, welche von Wiedergabeelementen der Wiedergabeeinrichtung in einer sensiblen Körperregion (zweite Körperregion) oder an einem sensiblen Körperteil (zweites Körperteil) wiedergegeben werden, können mit unterschiedlichen Vibrationsmustern wiedergegeben werden.

Vorzugsweise werden die Informationen zur Wiedergabe des taktilen Reizes drahtlos übertragen werden.

Anhand der nachfolgenden Figur soll die Erfindung beispielhaft näher erläutert werden.

Es zeigt:

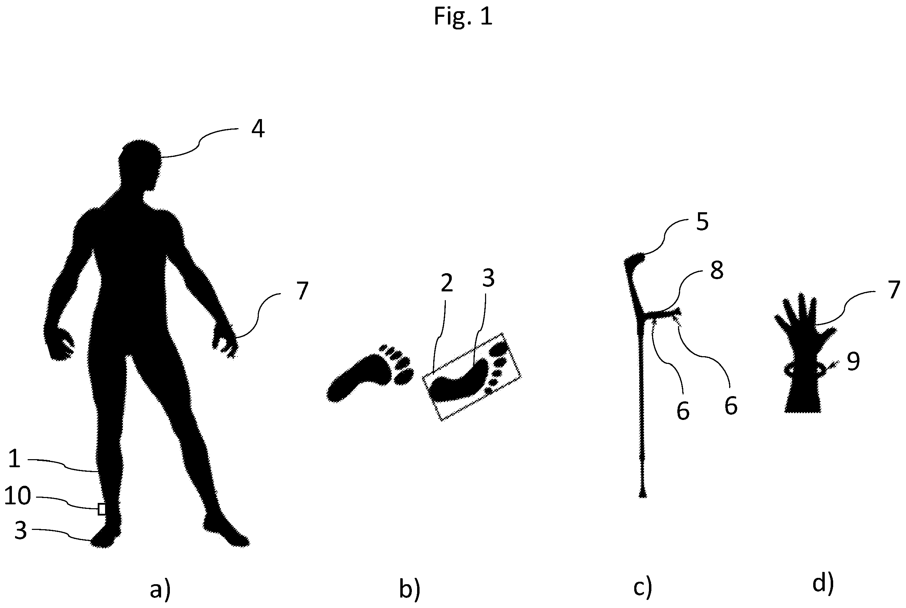

- Figur 1:: eine schematische Darstellung zur Erläuterung der Funktionsweise des orthopädischen Feedbacksystems

**Figur 1** zeigt eine schematische Darstellung zur Erläuterung der Funktionsweise des orthopädischen Feedbacksystems. Dabei zeigen Figur 1a eine Person 4, Figur 1b einen Fußabdruck eines Fußes 3 der Person 4, die Figur 1c eine Gehstütze 5 der Person 4 und Figur 1d eine Hand 7 der Person 4.

Das orthopädische Feedback-System weist eine mit dem Bein 1 als erstes Körperteil in Eingriff befindliches Stützelement (nicht gezeigt) auf, wobei es sich um eine Beinorthese handelt. Weiterhin weist das orthopädische Feedback-System eine mehrere Drucckrafterfassungssensoren aufweisende Druckkrafterfassungseinrichtung (nicht gezeigt) auf. Die Druckkrafterfassungssensoren sind in einer orthopädischen Einlegesohle 2 intrigiert, welche unter dem Fuß 3 der Person 4 angeordnet ist. Bei den Druckkrafterfassungssensoren handelt es sich um Force Sensitive Resistence (FSR) Sensoren, welche aus drei Schichten elektrischen Leiterbahnen ausgebildet sind. Zwischen der oberen elektrischen Leiterbahn und der unteren elektrischen Leiterbahn des FSR-Sensors befindet sich eine drucksensitive Substanz, welche ihren elektrischen Widerstand proportional zum Druck ändert. Mit zunehmendem Druck sinkt der elektrische Widerstand. Die Messung des elektrischen Widerstands erfolgt über die Messung der elektrischen Leitfähigkeit. Hierfür wird an einer oberen Leiterbahn eine Spannung angelegt und an der darunter befindlichen Leiterbahn die Spannung gemessen. Die Messung der Spannung wird an einem Referenzwiderstand, der zur Masse führt, am Eingang eines Analog-Digital-Wandlers (ADC) eins Mikrocontrollers (MCU) durchgeführt. Die Druckkrafterfassungseinrichtung kann eine in der Einlegesohle 2 integrierte Matrix mit 956 Druckerfassungssensoren aufweisen. Für die Weiterleitung der Signale der Druckerfassungssensoren wird ein Multiplexer eingesetzt. Die Druckkrafterfassungseinrichtung weist ferner eine Recheneinheit auf, mit welcher die Signale der Druckraftsensoren ausgewertet und in Informationen zur Wiedergabe von taktilen Reizen umgewandelt werden. Unter der gesamten Fußfläche des Fußes 3 werden somit kontinuierlich Druckwerte erfasst. Aus diesen Werten werden für den gewünschten Bereich unter Berücksichtigung vorgegebener Schwellwerte Informationen zur Wiedergabe von taktilen Reizen gebildet.

Weiterhin weist das orthopädische Feedback-System eine mit der Druckkrafterfassungseinrichtung in Bluetooth-Drahtloskommunikationsverbindung stehende Wiedergabeeinrichtung auf, welche in der Gehstütze 5 integriert ist. Dabei weist die Wiedergabeeinrichtung zwei Wiedergabeelemente 6 in Form von Vibrationsmotoren auf, welche zur taktilen Wiedergabe von mit der Drucckrafterfassungseinrichtung erfassten Druckkraftsignale an eine Hand 7 der Person 4 in einem Griff 8 der Gehstütze 5 integriert sind. Die Vibrationsmotoren 6 sind als Wiedergabeelemente derart in dem Griff 8 der Gehstütze 5 integriert, dass taktile Reize an die Hand 7 der Person 4 wiedergegeben werden können, wenn die Hand 7 den Griff 8 der Gehstütze 5 umgreift.

In Figur 1d ist die Wiedergabeeinrichtung in Form eines Armrings 9 ausgebildet, in welchen mindestens ein Vibrationsmotor als Wiedergabeelement ausgebildet ist. Der Armring wird von der Person 4 an einem Handgelenk der Hand 7 getragen, so dass taktile Reize zur Wiedergabe von mit der Druckkrafterfassungseinrichtung erfassten Druckkraftsignale am Handgelenk wahrgenommen werden können.

Gemäß eines Ausführungsbeispiels umfasst das Feedback-Verfahren folgende Schritte:
- Druckerfassung einer Druckkraftbelastung des Fußes 3 mit der in der orthopädischen Einlegesohle 2 integrierten Drucckrafterfassungseinrichtung,
- Weiterleiten der erfassten Druckkraftwerte an ein Sendemodul 10, welches im Bereich des Knöchels der Person 4 angeordnet ist,
- Funkübertagung der erfassten Druckkraftwerte mit dem Sendemodul 10 an ein mobiles Endgerät, beispielweise Smartphone, wobei erfassten Druckkraftwerte gespeichert werden,
- Funkübertagung der erfassten Druckkraftwerte mit dem Sendemodul 10 an eine Wiedergabeeinrichtung der Gehstütze 5 oder des Armrings 9, und
- Wiedergabe von auf den erfassten Druckkraftwerten basierenden taktilen Reizen mit den Wiedergabeeinrichtungen, so dass die Person 4 eine auf den Fuß 3 wirkende Druckkraftbelastung an der Handinnenfläche oder am Handgelenk wahrnehmen kann.

Infolge der taktilen Wiedergabe erhält die Person 4 einen Ersatzreiz für die fehlende Wahrnehmung des Fußes 3**.** Somit ist die Person 4 in der Lage, eine Muskulatur gezielt anzusteuern, um eine Unterbelastung oder Überbelastung des Fußes 3 durch Verlagerung der Körperposition zu kompensieren. Es hat sich gezeigt, dass die taktilen Reize, welche basierend auf die am Fuß 3 erfassten Druckkraftwerte im Bereich einer sensiblen Körperregionen wiedergegeben werden, als Empfindungen des Fußes wahrgenommen werden können.

### Bezugszeichenliste

| | |
|---|---|
| 1 | Bein |
| 2 | Einlegesohle |
| 3 | Fuß |
| 4 | Person |
| 5 | Gehstütze |
| 6 | Wiedergabeelemente / Vibrationsmotoren |
| 7 | Hand |
| 8 | Griff |
| 9 | Armring |
| 10 | Sendemodul |

## Patentansprüche

1. Orthopädisches Feedback-System aufweisend
mindestens eine als eine Orthese oder als eine Prothese ausgebildete orthopädische Stützvorrichtung, welche mindestens ein mit einem ersten Körperteil (1, 3) oder einer ersten Körperregion in Eingriff befindliches Stützelement aufweist,
eine mehrere Druckkrafterfassungssensoren aufweisende Drucckrafterfassungseinrichtung, welche zur Erfassung von einer auf das erste Körperteil (1, 3), die erste Körperregion oder das mindestens eine Stützelement wirkende Druckkraft eingerichtet ist, und
eine mit der Druckkrafterfassungseinrichtung in Kommunikationsverbindung stehende Wiedergabeeinrichtung, welche mindestens ein mit einem zweiten Körperteil (7) oder einer zweiten Körperregion kontaktiertes oder von dem zweiten Körperteil oder der zweiten Körperregion in Eingriff befindliches Wiedergabeelement (6) aufweist, welches zur taktilen Wiedergabe von mit der Druckkrafterfassungseinrichtung erfassten Drucckraftsignalen an das zweite Körperteil (7) oder die zweite Körperregion eingerichtet ist, **dadurch gekennzeichnet, dass** das Wiedergabeelement (6) eine mehrere beabstandet angeordnete Aktoren aufweisende Wiedergabematrix aufweist, welche eingerichtet ist, ein von der Druckkrafterfassungseinrichtung erfasstes Druckkraftprofil an dem zweiten Körperteil (7) oder die zweite Körperregion wiederzugeben.

2. Orthopädisches Feedback-System nach Anspruch 1, **dadurch gekennzeichnet, dass** das Wiedergabeelement (6) der Wiedergabeeinrichtung in eine separate Stützeinrichtung, welche sich von dem zweiten Körperteil (7) oder der zweiten Körperregion in Eingriff befindet, integriert ist.

3. Orthopädisches Feedback-System nach einem der Ansprüche 1 oder 2, **dadurch gekennzeichnet, dass** das Wiedergabeelement (6) der Wiedergabeeinrichtung in ein Kleidungstück, in einem Brustgurt, in einem Büstenhalter, in einem Armband, in einem Halsband oder Halskette, in einem Stirnband oder in einer Brille integriert ist.

4. Orthopädisches Feedback-System nach einem der Ansprüche 1 bis 3, **dadurch gekennzeichnet, dass** die Kommunikationsverbindung zwischen der Druckkrafterfassungseinrichtung und der Wiedergabeeinrichtung drahtlos ist.

5. Orthopädisches Feedback-System nach einem der Ansprüche 1 bis 4, **dadurch gekennzeichnet, dass** die Druckkrafterfassungseinrichtung in Form einer Einlegesohle (2) zur Einlage in einen Schuh, zur Einarbeitung auf eine orthopädische Einlage oder zur festen Verbindung mit dem Stützelement ausgebildet ist.

6. Orthopädisches Feedback-System nach einem der Ansprüche 1 bis 5, **dadurch gekennzeichnet, dass** Druckkrafterfassungssensoren an dem mindestens einen Stützelement angeordnet sind.

7. Orthopädisches Feedback-System nach einem der Ansprüche 1 bis 6, **dadurch gekennzeichnet, dass** die Druckkrafterfassungseinrichtung mindestens vier einzelne Druckkrafterfassungssensoren, bevorzugt mehr als 100 einzelne Druckkrafterfassungssensoren, besonders bevorzugt mehr als 1000 einzelne Druckkrafterfassungssensoren aufweist.

8. Feedback-Verfahren durchführbar mit dem orthopädischen Feedback-System nach einem der Ansprüche 1 bis 7, bei dem Verfahren eine an oder auf ein erstes Körperteil (1, 3), eine erste Körperregion und/oder ein Stützelement für das erste Körperteil (1, 3) oder die erste Körperregion wirkende Druckkraft an mehreren Druckkrafterfassungspositionen als Druckkraftprofil, Heat-Map-Diagramm oder topographisches Diagramm erfasst wird, wobei die Informationen des Druckkraftprofils, des Heat-Map-Diagramms oder des topographischen Diagramms mittels eines Wiedergabeelements (6) mit taktilen Reizen an mehreren Wiedergabepositionen an dem zweiten Körperteil (7) oder der zweiten Körperregion wiedergegeben werden.

9. Feedback-Verfahren nach Anspruch 8, **dadurch gekennzeichnet, dass** eine Intensität des taktilen Reizes an dem zweiten Körperteil (7) oder der zweiten Körperregion mit einer Intensität der an dem ersten Körperteil (1, 3) oder der ersten Körperregion erfassten Druckkraft korreliert.

10. Feedback-Verfahren nach einem der Ansprüche 8 oder 9,
**dadurch gekennzeichnet, dass** der taktile Reiz an dem zweiten Körperteil (7) oder der zweiten Körperregion bei Überschreiten und/oder Unterschreiten eines vorgegebenen Schwellenwertes einer erfassten Druckkraft wiedergegeben wird.

11. Feedback-Verfahren nach einem der Ansprüche 8 bis 10,
**dadurch gekennzeichnet, dass** bei Überschreiten oder Unterschreiten eines vorgegebenen Schwellenwertes einer erfassten Druckkraft ein vorgegebener taktiler Reiz wiedergegeben wird.

12. Orthopädisches Feedback-Verfahren nach einem der Ansprüche 8 bis 11, **dadurch gekennzeichnet, dass** der vorgegebene taktile Reiz als periodisches Vibrationssignal für eine vorgegebene Zeitdauer wiedergegeben wird.

13. Orthopädisches Feedback-Verfahren nach einem der Ansprüche 8 bis 12, **dadurch gekennzeichnet, dass** Informationen zur Wiedergabe des haptischen Feedback-Signals drahtlos übertragen werden.

## Claims

1. An orthopaedic feedback system having
at least one orthopaedic support apparatus in the form of an orthosis or prosthesis, which has at least one support element in engagement with a first body part (1, 3) or a first body region,
a compressive force detection device, which has multiple compressive force detection sensors and is configured to detect a compressive force acting on the first body part (1, 3), the first body region or the at least one support element, and
a reproduction device, which has a communications link with the compressive force detection device and has at least one reproduction element (6), which is in contact with a second body part (7) or a second body region or is in engagement with the second body part or the second body region and is configured for the tactile reproduction, on the second body part (7) or the second body region, of compressive force signals detected using the compressive force detection device, **characterised in that** the reproduction element (6) has a reproduction matrix, which has multiple spaced-apart actuators and is configured to reproduce, on the second body part (7) or the second body region, a compressive force profile detected by the compressive force detection device.

2. The orthopaedic feedback system according to Claim 1, **characterised in that** the reproduction element (6) of the reproduction device is integrated in a separate support device, which is in engagement with the second body part (7) or the second body region.

3. The orthopaedic feedback system according to one of Claims 1 or 2, **characterised in that** the reproduction element (6) of the reproduction device is integrated in an article of clothing, in a chest strap, in a bra, in an armband, in a neckband or necklace, in a headband or in a pair of spectacles.

4. The orthopaedic feedback system according to one of Claims 1 to 3, **characterised in that** the communications link between the compressive force detection device and the reproduction device is wireless.

5. The orthopaedic feedback system according to one of Claims 1 to 4, **characterised in that** the compressive force detection device is in the form of an insole (2) for inserting into a shoe, for incorporating onto an orthopaedic insert or for permanent connection to the support element.

6. The orthopaedic feedback system according to one of Claims 1 to 5, **characterised in that** compressive force detection sensors are arranged on the at least one support element.

7. The orthopaedic feedback system according to one of Claims 1 to 6, **characterised in that** the compressive force detection device has at least four individual compressive force detection sensors, preferably more than 100 individual compressive force detection sensors, particularly preferably more than 1000 individual compressive force detection sensors.

8. A feedback method that can be carried out using the orthopaedic feedback system according to one of Claims 1 to 7, in which method a compressive force acting on a first body part (1, 3), a first body region and/or a support element for the first body part (1, 3) or the first body region is detected at multiple compressive force detection positions as a compressive force profile, heat map diagram or topographical diagram, wherein the information of the compressive force profile, the heat map diagram or the topographical diagram is reproduced by means of a reproduction element (6) with tactile stimuli at multiple reproduction positions on the second body part (7) or the second body region.

9. The feedback method according to Claim 8, **characterised in that** an intensity of the tactile stimulus on the second body part (7) or the second body region correlates with an intensity of the compressive force detected on the first body part (1, 3) or the first body region.

10. The feedback method according to one of Claims 8 or 9, **characterised in that** the tactile stimulus is reproduced on the second body part (7) or the second body region when a detected compressive force exceeds and/or falls below a predefined threshold value.

11. The feedback method according to one of Claims 8 to 10, **characterised in that**, when a detected compressive force exceeds or falls below a predefined threshold value, a predefined tactile stimulus is reproduced.

12. The orthopaedic feedback method according to one of Claims 8 to 11, **characterised in that** the predefined tactile stimulus is reproduced as a periodic vibration signal for a predefined duration.

13. The orthopaedic feedback method according to one of Claims 8 to 12, **characterised in that** information for reproducing the haptic feedback signal is transmitted wirelessly.

## Revendications

1. Système de rétroaction orthopédique présentant
au moins un dispositif d'appui orthopédique réalisé sous la forme d'une orthèse ou d'une prothèse, qui présente au moins une béquille se trouvant en prise avec une première partie du corps (1, 3) ou une première région du corps,
un dispositif de détection de la force de pression présentant plusieurs capteurs de détection de la force de pression, qui est conçu pour détecter une force de pression agissant sur la première partie du corps (1, 3), la première région du corps ou l'au moins une béquille, et
un dispositif de reproduction se trouvant en liaison de communication avec le dispositif de détection de la force de pression, qui présente au moins un élément de reproduction (6) en contact avec une deuxième partie du corps (7) ou une deuxième région du corps ou en prise avec la deuxième partie du corps ou la deuxième région du corps, lequel élément de reproduction est conçu pour la reproduction tactile de signaux de force de pression détectés par le dispositif de détection de force de pression à la deuxième partie du corps (7) ou à la deuxième région du corps, **caractérisé en ce que** l'élément de reproduction (6) présente une matrice de reproduction comportant plusieurs actionneurs disposés à distance, qui est conçue pour reproduire un profil de force de pression détecté par le dispositif de détection de force de pression sur la deuxième partie du corps (7) ou la deuxième région du corps.

2. Système de rétroaction orthopédique selon la revendication 1, **caractérisé en ce que** l'élément de reproduction (6) du dispositif de reproduction est intégré dans un dispositif d'appui séparé qui est engagé par la deuxième partie du corps (7) ou la deuxième région du corps.

3. Système de rétroaction orthopédique selon l'une des revendications 1 ou 2, **caractérisé en ce que** l'élément de reproduction (6) du dispositif de reproduction est intégré dans un vêtement, dans une ceinture pectorale, dans un soutien-gorge, dans un bracelet, dans un collier ou une chaîne de cou, dans un bandeau frontal ou dans des lunettes.

4. Système de rétroaction orthopédique selon l'une des revendications 1 à 3, **caractérisé en ce que** la liaison de communication entre le dispositif de détection de la force de pression et le dispositif de reproduction est sans fil.

5. Système de rétroaction orthopédique selon l'une des revendications 1 à 4, **caractérisé en ce que** le dispositif de détection de la force de pression se présente sous la forme d'une semelle intérieure (2) destinée à être insérée dans une chaussure, à être intégrée à une semelle orthopédique ou à être reliée de manière fixe à la béquille.

6. Système de rétroaction orthopédique selon l'une des revendications 1 à 5, **caractérisé en ce que** des capteurs de détection de la force de pression sont disposés sur ladite au moins une béquille.

7. Système de rétroaction orthopédique selon l'une des revendications 1 à 6, **caractérisé en ce que** le dispositif de détection de la force de pression comporte au moins quatre capteurs individuels de détection de la force de pression, de préférence plus de 100 capteurs individuels de détection de la force de pression, de manière particulièrement préférée plus de 1000 capteurs individuels de détection de la force de pression.

8. Procédé de rétroaction réalisable avec le système de rétroaction orthopédique selon l'une des revendications 1 à 7, dans lequel procédé une force de pression agissant à ou sur une première partie du corps (1, 3), une première région du corps et/ou une béquille pour la première partie du corps (1, 3) ou la première région du corps est saisie à plusieurs positions de détection de la force de pression sous forme de profil de force de pression, de diagramme de carte de chaleur ou de diagramme topographique, les informations du profil de force de pression, du diagramme de carte de chaleur ou du diagramme topographique étant reproduites au moyen d'un élément de reproduction (6) avec des stimuli tactiles à plusieurs positions de reproduction sur la deuxième partie du corps (7) ou la deuxième région du corps.

9. Procédé de rétroaction selon la revendication 8, **caractérisé en ce qu'**une intensité du stimulus tactile sur la deuxième partie du corps (7) ou la deuxième région du corps est en corrélation avec une intensité de la force de pression détectée sur la première partie du corps (1, 3) ou la première région du corps.

10. Procédé de rétroaction selon l'une des revendications 8 ou 9, **caractérisé en ce que** le stimulus tactile est reproduit sur la deuxième partie du corps (7) ou la deuxième région du corps en cas de dépassement et/ou de sous-dépassement d'une valeur seuil prédéfinie d'une force de pression détectée.

11. Procédé de rétroaction selon l'une des revendications 8 à 10, **caractérisé en ce qu'**un stimulus tactile prédéfini est reproduit en cas de dépassement ou de sous-dépassement d'une valeur seuil prédéfinie d'une force de pression détectée.

12. Procédé de rétroaction orthopédique selon l'une des revendications 8 à 11, **caractérisé en ce que** le stimulus tactile prédéfini est reproduit sous la forme d'un signal de vibration périodique pendant une durée prédéfinie.

13. Procédé de rétroaction orthopédique selon l'une des revendications 8 à 12, **caractérisé en ce que** des informations pour la reproduction du signal de rétroaction haptique sont transmises sans fil.
